**Europäisches Patentamt**

**European Patent Office**

**Office européen des brevets**

(11) Veröffentlichungsnummer: **0 419 759 B1**

(12) # EUROPÄISCHE PATENTSCHRIFT

(45) Veröffentlichungstag der Patentschrift: **07.09.94**

(51) Int. Cl.⁵: **C07C 53/126**, A61K 7/00, A61K 31/19

(21) Anmeldenummer: **90108297.4**

(22) Anmeldetag: **02.05.90**

(54) **Aluminium, Magnesium-Hydroxi-Fettsäureverbindungen und damit hergestellte thermostabile Lipogele.**

(30) Priorität: **28.09.89 DE 3932377**

(43) Veröffentlichungstag der Anmeldung:
**03.04.91 Patentblatt 91/14**

(45) Bekanntmachung des Hinweises auf die
Patenterteilung:
**07.09.94 Patentblatt 94/36**

(84) Benannte Vertragsstaaten:
**DE ES FR GB IT**

(56) Entgegenhaltungen:
**EP-A- 0 308 646**
**EP-A- 0 318 642**

(73) Patentinhaber: **Giulini Chemie GmbH**
**Giulinistrasse 2**
**Postfach 150 480**
**D-67029 Ludwigshafen (DE)**

(72) Erfinder: **Assmus, Uwe, Dipl.-Ing.**
**Bahnhofstrasse 29**
**D-6107 Reinheim 1 (DE)**
Erfinder: **Schanz, Klaus, Dr.**
**In der Zeil 5**
**D-6701 Dannstadt-Schauernheim (DE)**
Erfinder: **Kaufmann, Bruno**
**Ormsheimerhof 16**
**D-6701 Frankenthal (DE)**

**Beschreibung**

Die vorliegende Erfindung betrifft thermostabile Lipogele, die eine neue Aluminium, Magnesiumhydroxi-Fettsäure Verbindung und eine organisch- lipophile Verbindung enthalten, ferner die Herstellung der Aluminium,Magnesiumhydroxi-Fettsäure Verbindung und der Lipogele, sowie die Verwendung der Lipogele in kosmetischen und pharmazeutischen Zubereitungen.

Bekannt sind Gelzusammensetzungen auf der Basis von Bentoniten und Montmorriloniten, sowie von sog. organisch modifizierten Bentoniten, siehe dazu Jordan, J. W. Jour. Phys. and Colloid Chem., 53, 294 (1949) oder EP 0 204 240 und DE-OS 31 45 449.

Diese organisch modifizierten Tonmineralien zeigen in Ölen, Fetten und Wachsen eine gute Quellwirkung und bilden mit ihnen unter Anwendung von mechanischer Energie , geeigneter Additive und geeigneter Temperatur viskose Gele.

Ein großer Nachteil dieser bekannten Gelzubereitungen besteht darin, daß sie recht große Mengen an organisch- polaren Additiven, wie Methanol, Aceton etc. und die durch den Ionenaustausch eingebrachten quartären Ammoniumsalze enthalten, die unter Umständen eine hautreizende Wirkung entfalten können und toxikologisch nicht ganz unbedenklich sind, und außerdem eine braune bis beige Eigenfarbe aufweisen, die in kosmetischen Formulierungen von Nachteil ist.

In der DE-OS 37 26 265, die von der Anmelderin stammt, wurden neue Aluminium, Magnesium-Hydroxi-Verbindungen, die gelbildende Eigenschaften haben vorgeschlagen. Diese Verbindungen unterscheiden sich von den bekannten organisch modifizierten Bentoniten, sowohl in ihrer Struktur, als auch in ihrer Zusammensetzung. Die Struktur dieser A1. Mg-Hydroxi-Verbindungen leitet sich vom Brucit $Mg(OH)_2$ ab, bei dem die $OH^-$ Ionen eine hexagonaldichteste Kugelpackung bilden und die oktaedrischen Lücken mit $Mg^2+$ Kationen besetzt werden. Die Schichtstruktur ergibt sich dadurch, daß nur jede zweite Schicht der oktaedrischen Lücken von Metallatomen besetzt wird.

Der Ersatz von zweiwertigen Mg-Kationen durch dreiwertiges Aluminium führt zu einer positiven Überschußladung in den Oktaederschichten, die durch Anionen in der Zwischenschicht ausgeglichen werden - übrigens im Gegensatz zu den organisch-modifizierten Bentoniten, bei denen die Zwischenschicht positiv geladene quartäre Ammoniumgruppen zum Ladungsausgleich enthält.

Die Aluminium, Magnesium-Hydroxi-Verbindungen gemäß der DE-OS 37 26 265 bilden, wie schon erwähnt, mit vielen organisch-lipophilen Verbindungen Gele, die im Vergleich zu den Gelen, die mit organisch-modifizierten Bentoniten gebildet werden, eine absolut weiße Frabe aufweisen. Diese Gele eignen sich natürlich sehr gut für kosmetische Formulierungen, bei denen es auf eine reine weiße Farbe besonders ankommt.

Diese enthalten auch Aluminium, Magnesium-Hydroxi-Verbindungen keine quartären Ammoniumgruppen und sehr wenig polare Additive und sind deshalb weniger hautreizend, als die entsprechenden Formulierungen auf der Basis der organisch -modifizierten Bentonite.

Die Herstellung der entsprechenden Gele war jedoch nicht unproblematisch, denn sie erforderte den Einsatz von anspruchsvollen Rühraggreagaten, die hohe Scherkräfte erzeugen konnten, dazu siehe die DE-OS 37 32 265.

Bei den Gelen, hergestellt gemäß der DE-OS 37 32 265, kam es auch häufiger vor, daß sie gegen Temperatureinflüsse nicht stabil genug waren. In einem Wärmestreßtest z.B. bei ca. 50 Grad C zeigten diese Gele eine leichte Ölseparation. Die Viskosität der Gele veränderte sich entsprechend, sie wurden deutlich dünnflüssiger und ebenso die damit hergestellten kosmetischen Formulierungen.

Eine solche deutliche Ölseparation kann jedoch in kosmetischen Formulierungen nicht immer akzeptiert werden - sie ist außerdem unwirtschaftlich, sollten diese Gele doch auch die Fertigformulierung stabilisieren und gegebenenfalls zusätzlich noch Öl binden.

Es stellte sich daher die Aufgabe eine verbesserte Gelzubereitung zu finden, die folgenden Anforderungen gerecht wird :

1. keine Ölseparation unter Temperatureinfluß
2. ausreichend hohe Viskosität auch bei höheren Lagertemperaturen(z. B. 50°C) und bei Lagerzeiten von mindestens 3 Monaten
3. leichte, reproduzierbare und ökonomische Herstellbarkeit
4. enthält keine polaren Additive

Die gestellte Aufgabe konnte überraschenderweise durch eine Gelzubereitung gelöst werden, die als gelbildende Komponente eine neue Aluminium, Magnesiumhydroxi-Fettsäure Verbindung und eine bei Raumtemperatur (20 Grad C) flüssige, organische lipophile Verbindung enthält.

Die in die Gelzubereitungen eingearbeitete neue Aluminium, Magnesiumhydroxi-Fettsäure Verbindung weist eine Schichtstruktur auf und besitzt die allgemeinen Formel:

$$Al_2 Mg_y(OH)_{4+2y}R_2 \cdot n\ H_2O$$

in der R den Rest einer aliphatischen Monocarbonsäure R'COO⁻ und deren Mischungen darstellt, wobei R'COO⁻ 12 bis 22 Kohlenstoffatome enthält und y folgende Bedingung erfüllt:

$2 \leq y \leq 10$ und n eine ganze Zahl größer Null ist.

Die neuen Verbindungen sind durch die Umsetzung eines löslichen Aluminiumsalzes mit einem löslichen Magnesiumsalz im alkalischen Milieu bei einem pH-Wert ≥ 8 und in Gegenwart eines Alkali- oder Ammoniumsalzes einer 12 bis 22 Kohlenstoffatome enthaltenden aliphatischen Monocarbonsäure und deren Mischungen erhältlich , wobei die wässerigen Lösungen der Aluminium-und Magnesiumsalze gleichzeitig in die alkalische Alkalicarbonsäurelösung bei Temperaturen von 20 bis 90 Grad C, bevorzugt 60 bis 85 Grad C unter intensivem Rühren zugegeben werden. Der pH-Wert muß stets ≥ 8 betragen. Der so erhaltene Niederschlag wird nach bekannten Methoden abgetrennt und mit vollentsalztem Wasser solange gewaschen, bis im Waschwasser keine Anionen der jeweils eingesetzten Aluminium und Magnesiumsalze mehr nachweisbar sind. Es ist von besonderer Wichtigkeit die Umsetzung unter Ausschluß von Kohlendioxid und Härtebildnern bzw. anderen Fremdionen, d.h. unter Verwendung von vollentsalztem Wasser, durchzuführen.

Als wasserlösliche Aluminiumverbindung, die für diese Umsetzung geeignet sind, kommen die Halogenide, Nitrate und Sulfate des Aluminiums , sowie die basischen Aluminiumhalogenide, z. B. $Al_2(OH)_5Cl$ (ACH), und auch Alkalialuminate in Frage. Die entsprechenden Magnesiumverbindungen umfassen die Magnesiumhalogenide-, nitrate- und sulfate. Die Einstellung des pH-Wertes während der Umsetzung sollte stets mit Alkalihydroxiden oder mit wässerigem Ammoniumhydroxid erfolgen. Bei jedem Verfahrensschritt sollte aus oben genannten Gründen auf die Abwesenheit von Kohlendioxid geachtet werden. Es ist daher empfehlenswert, die Umsetzung in einem geschlossenen Reaktor unter Inertgas durchzuführen.

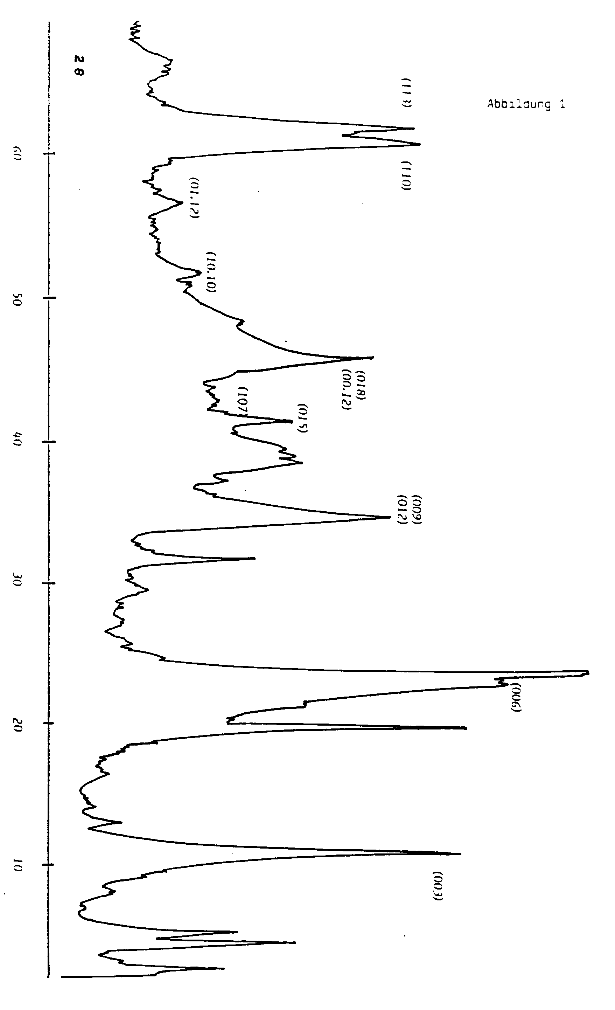

Die neuen Aluminium, Magnesiumhydroxi-Fettsäure Verbindungen sind feinkristalline, weiße puverförmige Stoffe. Ihre Charakterisierung, insbesondere der Nachweis ihrer Schichtstruktur kann mit Hilfe des Röntgenbeugungsspektrums erbracht werden, siehe Röntgenbeugungsspektrum auf Abb. 1 Tabelle 5.

Die 12 bis 22 Kohlenstoffatome enthaltenden organischen Monocarbonsäuren umfassen die Verbindungen mit der allg. Summenformel $C_nH_{2n+1}COOH$, also solche wie z.B. Myristinsäure, Palmitinsäure, Stearinsäure etc. und auch ihre Mischungen z.B. die Mischung der Palmitin- und Stearinsäuren (C16-C18 Mischung). Diese Säuren sind im Handel erhältlich oder können nach Bedarf gemischt und ohne Vorreinigung eingesetzt werden.

Die neuen Aluminium, Magnesiumhydroxi-Fettsäure Verbindungen werden durch Vakuumtrocknung oder Sprühtrocknung isoliert. Als Ausgangsstoff für die Gelherstellung kann sowohl der Filterkuchen nach dem Waschen, als auch der in Wasser resuspendierte, sprühgetrocknete Filterkuchen verwendet werden.

Die Gelherstellung erfolgt in einem geschlossenen Reaktor mit Rühreinrichtung, wobei man auf aufwendige, Scherkräfte erzeugende Rührer verzichten kann. Die Umsetzung verläuft quantitativ bei Temperaturen zwischen 90 und 130 Grad C unter intensivem Rühren der beiden Komponenten und dem sich einstellenden Dampfdruck, der neuen Aluminium,Magnesiumhydroxi-Fettsäure Verbindung und der organisch-lipophilen Verbindung.

Als organisch-lipophile Verbindung, die die Gelbildung mit der neuen Al, Magnesiumhydroxi-Fettsäure Verbindung ermöglicht, kann mindestens eine Verbindung aus den folgenden Gruppen eingesetzt werden:

a. pflanzliche und tierische Fette und Öle, z.B. Lanolin, Jojobaöl, Rizinusöl,

b. Paraffinkohlenwasserstoffe, z. B. Mineralöl, Isoparaffine,

c. Silikonöle, z. B. Cyclomethicone (tetramer oder pentamer),

d. aliphatische und aromatische Ester, z. B. Isopropylmyristat, Isopropylpalmitat,

c. höhere Alkohole und Ether, z. B. 2-Octyl-Dodecanol.

Die Konzentration der Aluminium, Magnesiumhydroxi-Fettsäure Verbindung beträgt 10 bis 30 Gew. %, bevorzugt 20 Gew. % , bezogen auf das Lipogel, während die der organisch-lipophilen Verbindung zwischen 90 bis 70 Gew. %, bevorzugt 80 Gew. %,liegt.

Das so hergestellte Lipogel ist ein farbloses, hochviskoses Gel, das unter Wärmeeinwirkung besonders viskositätsstabil bleibt. Besonders die damit hergestellten kosmetischen Erzeugnisse zeigen keinerlei Ölseparation.

Die nachfolgenden Beispiele illustrieren zunächst die Herstellung der neuen Aluminium, Magnesiumhydroxi-Fettsäure Verbindungen:

3

Beispiel 1

Herstellung von Al,Magnesiumhydroxi-Stearat/Palmitat

$Al_2 Mg_6 (OH)_{16} (C_{17}H_{35}COO)_{1,3} (C_{15}H_{31}COO)_{0,7}$

In einem geschlossenen Reaktor mit Inertgas werden 182,1 kg vollentsalztes Wasser vorgelegt und 8,73 kg eines handelsüblichen Na-Stearats (65 % C18/35 % C16 ) suspendiert. Anschließend heizt man bei laufendem Rührwerk auf 80 Grad C, bis eine klare Lösung entsteht. In einem separaten Rührbehälter werden 29,55 kg vollentsalztes Wasser vorgelegt und nacheinander 7,11 kg $AlCl_3$ x 6 $H_2O$ und 17,94 kg $MgCl_2$x6 $H_2O$ gelöst.

Die klare Lösung von $AlCl_3/MgCl_2$, sowie 54,6 kg einer 17,3 %igen NaOH Lösung dosiert man parallel in das Vorlagegefäß derart, daß der pH-Wert stets größer als 8 eingehalten wird. Nach ca. 1 Stunde Rührzeit hat sich der pH-Wert von 9,8 eingestellt. Man kühlt die Suspension auf Raumtemperatur ab und filtriert das unlösliche Al,Magnesiumhydroxi-Stearat/Palmitat ab.

Mit vollentsalztem Wasser wird solange gewaschen, bis kein Chlorid mehr mit $AgNO_3$ Lösung nachweisbar ist. Anschließend trocknet man den Filterkuchen mit den üblichen Einrichtungen (Sprühtrockner, Vakuumtrockner usw. )

| | |
|---|---|
| Ausbeute: | 13,8 kg (92 %) |
| Beschreibung: | weißes, feinkristallines Pulver |
| Chemische Analyse: | 5,31 % Al i.Tr. (theoretisch 5,29 %) |
| | 14,34 %Mg i.Tr. (theoretisch 14,31 %) |
| | 54,02 % $C_{17}H_{35}$COOH/ $C_{15}H_{31}$COOH i.Tr.(theoretisch 53,7 %) |

Beispiel 2:

Herstellung von Al, Magnesiumhydroxi-Stearat

$Al_2 Mg_6 (OH)_{16} (C_{17}H_{35}COO)_2$

In einem geschlossenen Reaktor mit Inertgas werden 108,71 kg vollentsalztes Wasser vorgelegt und unter Rühren 4,11 kg Stearinsäure (98 % $C_{18}$) suspendiert. Anschließend gibt man 10,40 kg NaOH 50 %ig dazu und heizt auf 80°C bei laufendem Rührwerk. In einem separaten Behälter werden 14,48 kg vollentsalztes Wasser vorgelegt und nacheinander 3,49 kg $AlCl_3$ x 6$H_2O$ und 8,81 kg $MgCl_2$x6$H_2O$ gelöst. Die klare Lösung wird innerhalb von 30 Minuten unter intensivem Rühren in das Vorlagegefäß dosiert. Nach weiteren 30 Minuten Rührzeit hat sich ein pH-Wert von 9,5 eingestellt. Man kühlt die Suspension auf Raumtemperatur und filtriert das unlösliche Al, Magnesiumhydroxi-Stearat ab.

Mit vollentsalztem Wasser wird solange gewaschen, bis kein Chlorid mehr mit $AgNO_3$-Lösung als AgCl nachweisbar ist. Anschließend trocknet man den Filterkuchen mit den üblichen Einrichtungen (Sprühtrockner, Vakuumtrockner usw.).

| | |
|---|---|
| Ausbeute: | 7,1 kg (94 %) |
| Beschreibung: | weißes, feinkristallines Pulver |
| Chem. Analyse: | 5,23 % Al i. Tr. (theoretisch 5,19 %) |
| | 14,13 % Mg i. Tr. (theoretisch 14,04 %) |

54,80 % $C_{17}H_{35}$COOH i. Tr. (theoretisch 54,57 %)

Beispiel 3:

Herstellung von Al, Magnesiumhydroxi-Behenat

$Al_2 Mg_4 (OH)_{12} (C_{21}H_{43}COO)_2$

In einem geschlossenen Reaktor mit Inertgas werden 64,92 kg vollentsalztes Wasser vorgelegt und unter Rühren 3,28 kg Behensäure (98 % $C_{22}$) suspendiert. Anschließend gibt man 7,66 kg KOH 50 %ig dazu und heizt auf 80°C bei laufendem Rührwerk. In einem separaten Behälter werden 17,88 kg vollentsalztes Wasser vorgelegt und nacheinander 2,33 kg $AlCl_3$ x 6$H_2O$) und 3,93 kg $MgCl_2$ x 6 $H_2O$ gelöst. Die klare Lösung wird innerhalb von 30 Minuten unter intensivem Rühren in das Vorlagegefäß dosiert. Nach weiteren 30 Minuten Rührzeit hat sich ein pH-Wert von 10 eingestellt. Man kühlt die Suspension auf Raumtemperatur und filtriert das unlösliche Al, Magnesiumhydroxi-Behenat ab.

Mit vollentsalztem Wasser wird solange gewaschen, bis kein Chlorid mehr mit $AgNO_3$-Lösung als AgCl nachweisbar ist. Anschließend trocknet man den Filterkuchen mit den üblichen Einrichtungen (Sprühtrockner, Vakuumtrockner usw.).

Ausbeute: 4,8 kg (96 %)

Beschreibung: weißes, feinkristallines Pulver

Chem. Analyse: 5,18 % Al i. Tr. (theoretisch 5,22 %)
9,35 % Mg i. Tr. (theoretisch 9,40 %)
65,52 % $C_{21}H_{43}COOH$ i. Tr. (theoretisch 65,66 %)

Beispiel 4:

Herstellung von Al, Magnesiumhydroxi-Myristat

$Al_2Mg_4(OH)_{12}(C_{13}H_{27}COO)_2$

In einem geschlossenen Reaktor mit Inertgas werden 60 kg vollentsalztes Wasser vorgelegt und unter Rühren 2,81 kg Myristinsäure (98 % $C_{14}$) suspendiert. Anschließend gibt man 9,67 kg KOH 50 %ig dazu und heizt auf 80°C bei laufendem Rührwerk. In einem separaten Behälter werden 17,33 kg vollentsalztes Wasser vorgelegt und nacheinander 4,11 kg $Al_2(SO_4)_3$ x 18 $H_2O$ und 6 kg $MgSO_4$ x 7 $H_2O$ gelöst. Die klare Lösung wird innerhalb von 30 Minuten unter intensivem Rühren in das Vorlagegefäß dosiert. Nach weiteren 30 Minuten Rührzeit hat sich ein pH-Wert von 10 eingestellt. Man kühlt die Suspension auf Raumtemperatur und filtriert das unlösliche Al, Magnesiumhydroxi-Myristat ab. Mit vollentsalztem Wasser wird solange gewaschen, bis kein Sulfat mehr mit $BaCl_2$-Lösung als $BaSO_4$ nachweisbar ist. Anschließend trocknet man den Filterkuchen mit den üblichen Einrichtungen (Sprühtrockner, Vakuumtrockner usw.).

Ausbeute: 4,7 kg (94 %)

Beschreibung: weißes, feinkristallines Pulver

Chem. Analyse: 6,61 % Al i. Tr. (theoretisch 6,66 %)
11,94 % Mg i. Tr. (theoretisch 12,00 %)
56,08 % $C_{13}H_{27}COOH$ i. Tr. (theoretisch 56,14 %)

Die folgenden Beispiele 5 bis 8 beschreiben die Herstellung der Lipogele unter Verwendung der neuen Al, Magnesiumhydroxi-Fettsäure- Verbindung:

Beispiel 5:

Herstellung eines Cyclomethicone-Lipogels

In einem geschlossenen 1 $m^3$ Mischer (Typ Becomix, Bremen) werden 236 kg Cyclomethicone pentamer (incl. 5 % Verdunstungsverluste) und 83 kg eines vakuumgetrockneten Al,Magnesiumhydroxi-Stearat Pulvers (mit 10 % Feuchte) aus Beispiel 2 eingewogen und innerhalb von 30 Minuten unter Kräftigem Rühren homogen suspendiert. Anschließend erhitzt man auf 110°C, wobei sich ein Dampfdruck von ca. 2,0 bar einstellt. Diese Bedingungen werden 30 Minuten gehalten. Anschließend wird Vakuum gezogen, wobei dieses so reguliert werden sollte, daß die Temperatur nicht wesentlich unter 105°C sinkt. Der Vorgang ist beendet, wenn das Wasser vollständig ausgedampft ist, d.h. unter Vakuum stellt sich konstant eine Temperatur von 110°C ein. Das während der Gelherstellung ausgedampfte Wasser/Cyclomethicone-Gemisch wird kondensiert, getrennt und so kann das Cyclomethicone in einer weiteren Umsetzung wiederverwendet werden.

Danach kühlt man langsam auf 80°C und füllt das Produkt in Fässer ab. In diesen Behältnissen kühlt das fertige Gel auf Raumtemperatur ab.

Ausbeute: 261 kg (87 %) eines weißen Cyclomethicone-Lipogels.

Beispiel 6:

Herstellung eines Isopropylpalmitat-Lipogels

In einem geschlossenen 1 $m^3$ Mischer (Typ Becomix, Bremen) werden 225 kg Isopropylpalmitat und 88 kg eines vakuumgetrockneten Al, Mg-hydroximyristatpulvers (mit 15 % Feuchte) aus Beispiel 4 eingewogen und innerhalb von 30 Minuten unter kräftigem Rühren homogen suspendiert. Anschließend erhitzt man auf 120°C, wobei sich durch den Wasserdampf ein Druck von ca. 1,6 bar einstellt. Diese Bedingungen werden 30 Minuten gehalten. Anschließend wird langsam Vakuum gezogen, wobei dieses so reguliert werden sollte, daß die Temperatur nicht wesentlich unter 110°C sinkt. Der Vorgang ist beendet, wenn das Wasser vollständig ausgedampft ist, d.h. unter Vakuum stellt sich konstant eine Temperatur von 120°C ein. Danach kühlt man langsam auf 100°C und füllt das Produkt in Fässer ab. In diesen Behältnissen kühlt das fertige Gel auf Raumtemperatur ab.

Ausbeute:  269 kg (90 %) eines weißen Isopropylpalmitat-Lipogels.

Beispiel 7:

Herstellung eines Isopropylmyristat-Lipogels

In einem geschlossenen Labormischer (maximaler Inhalt 5 kg) werden 3200 g Isopropylmyristat und 2.286 g Filterkuchen von Al, Magnesiumhydroxi-Stearat/Palmitat (mit 65 % Feuchte) aus Beispiel 1 eingewogen und innerhalb von 30 Minuten unter kräftigem Rühren homogen suspendiert. Anschließend erhitzt man auf 120°C, wobei sich durch den Wasserdampf ein Druck von ca, 1,8 bar einstellt. Diese Bedingungen werden 30 Minuten gehalten. Anschließend wird langsam Vakuum gezogen, wobei dieses so reguliert werden sollte, daß die Temperatur nicht wesentlich unter 110°C sinkt. Der Vorgang ist beendet, wenn das Wasser vollständig ausgedampft ist, d.h. unter Vakuum stellt sich konstant eine Temperatur von 120°C ein. Danach kühlt man langsam innerhalb von 2 Stunden auf Raumtemperatur ab.

Ausbeute:  3975 g (99 %) eines weißen Ispropylmyristat-Lipogels.

Beispiel 8:

Herstellung eines Paraffinöl-Lipogels

In einem geschlossenen Labormischer (maximaler Inhalt 5 kg) werden 1600 g Paraffinum subliquidum und 430 g des sprühgetrockneten Al, Magnesiumhydroxi-Stearates (mit 7 % Feuchte) aus Beispiel 2 eingewogen und innerhalb von 10 Minuten unter kräftigem Rühren homogen suspendiert. Anschließend gibt man 40 g vollentsalztes Wasser dazu, rührt nochmals 20 min. weiter und erhitzt auf 120°C. Durch den Wasserdampf stellt sich ein Druck von ca, 1,6 bar ein. Diese Bedingungen werden 30 Minuten gehalten. Anschließend wird langsam Vakuum gezogen, wobei dieses so reguliert werden sollte, daß die Temperatur nicht wesentlich unter 110°C sinkt. Der Vorgang ist beendet, wenn das Wasser vollständig ausgedampft ist, d.h. unter Vakuum stellt sich konstant eine Temperatur von 120°C ein. Danach kühlt man langsam innerhalb von 1 Stunde auf Raumtemperatur ab.

Ausbeute:  1980 g (99 %) eines farblosen, leicht transparenten Paraffinöl-Lipogels

In den folgenden Beispielen wird die stark verbesserte Thermostabilität der neuen Lipogele ,sowie einige charakteristischen Eigenschaften, z.B. ihre Eignung als Suspendierhilfsmittel, gezeigt.

Beispiel 9:

Überprüfung der Konsistenzstabilität von Al,Magnesiumhydroxi-Fettsäure Lipogelen

Die Konsistenz der Gele wird über die Mikrokonuspenetration nach Klein bei verschiedenen Temperaturen bestimmt.

Tabelle 1

| | Mikropenetration (0.1 mm) | | |
|---|---|---|---|
| | 25 °C | 40 °C | 60 °C |
| Gel aus Beispiel 8 | 25 | 28 | 38 |
| Gel aus Beispiel 7 | 31 | 34 | 42 |
| Gel aus Beispiel 6 | 35 | 38 | 42 |
| Gel aus Beispiel 5 | 40 | 41 | 46 |
| Paraffinöl-Bentonit-Gel | 74 | 96 | 109 |
| Isopropylmyristat-Bentonit-Gel | 60 | 65 | 84 |

Wie man anhand der obigen Tabelle 1 erkennen kann, treten bei den getesteten Temperaturen überraschenderweise kaum Konsistenzänderungen auf. Im Gegensatz zu den bisher bekannten organisch-modifizierten Bentonit-Gelen sind die Lipogele gemäß den Beispielen 5 bis 8 schon bei Raumtemperatur deutlich fester und bei höheren Lagertemperaturen wesentlich viskositätsstabiler. Dies ist ein ganz wichtiger Aspekt bei der Herstellung oder Lagerung bestimmter Kosmetikprodukte . So verflüssigen sich z. B. herkömmliche Sonnenschutzformulierungen bei den üblichen sommerlichen Außentemperaturen, oder beim Gießen von Lippenstiften (bei ca. 60 Grad C) sedimentieren die Farbpigmente. Sind jedoch rheologische Additive, wie z. B. Gele aus den Beispielen 5 bis 8 enthalten, kann die Viskosität und Konsistenz der Produkte auch bei erhöhten Temperaturen konstant gehalten werden.

Beispiel 10:

Überprüfung der Lagerstabilität bei erhöhter Temperatur von Al, Magnesiumhydroxi-Fettsäure Lipogelen

Die Lagerstabilität erfolgte durch visuelle Beurteilung der Ölseparation bei verschiedenen Temperaturen im Klimaschrank.

Beurteilungsparameter: x = stabil, xx = beginnende Separation, xxx = deutliche Separation

Tabelle 2

| | Mikropenetration (0,1 mm) | | 3 Mon. bei Raumtemp. | 3 Mon. bei 50 °C |
|---|---|---|---|---|
| | bei Lagerbeginn | nach 3 Mon. Lagerung bei 50 °C | | |
| Gel aus Beisp. 8 | 25 | 29 | x | x |
| Gel aus Beisp. 7 | 31 | 36 | x | x |
| Gel aus Beisp. 6 | 35 | 40 | x | x |
| Gel aus Beisp. 5 | 40 | 43 | x | xx |

Die beginnende Separation des Gels aus Beispiel 5 nach 3 Monaten bei 50 °C ist durch die Verdunstung des flüchtigen Silikonöls zu erklären, ansonsten ist bei keinem der Gele eine Ölseparation zu beobachten. Die Lipogele aus den Beispielen 5 bis 8 zeigen im Vergleich zu den bisher bekannten Gelen, z. B. gemäß DE-OS 37 32 265 eine nochmalige Verbesserung, auch bezüglich der Stabilität. Wie bereits aus der Tabelle 14 der DE-OS 37 32 265 bekannt ist, zeigen die organisch modifizierten Bentonitgele nach 1 Woche Lagerung bei 50 Grad C eine deutliche Ölseparation. Dagegen zeigten die aus DE-OS 37 32 265 hergestellten Gele nach 1 Woche und auch nach einem Monat noch keinerlei Veränderungen. In einem sog. Wärmestreßtest, der in der Kosmetikindustrie üblich ist, zeigen auch diese Gele nach etwa drei Monaten bei 50 Grad C eine leichte Ölseparation. Die Gele gemäß der vorliegenden Erfindung aus den Beispielen 5 bis 8 sind jedoch absolut stabil.

Beispiel 11:

Überprüfung des Absetzverhaltens in einer Antiperspirant-Aerosol-Formulierung

Um die Wirksamkeit der Lipogele als Antiabsetzmittel in feststoffhaltigen Aerosolen zu überprüfen, wurden folgende Antiperspirant-Formulierungen hergestellt:

Tabelle 3

|  | A | B | C | D |
|---|---|---|---|---|
| Gel aus Beispiel 5 | 7,25 | - | - | - |
| Gel aus Beispiel 7 | - | 7,25 | - | - |
| Siliciumdioxid | - | - | 1,00 | - |
| Cyclomethicone pentamer | 4,50 | 4,50 | 4,50 | 4,50 |
| Isopropylmyristat | 5,00 | 5,00 | 11,25 | 12,25 |
| Aluminiumchlorhydrat Pulver | 8,25 | 8,25 | 8,25 | 8,25 |
| n-Pentan | 40,00 | 40,00 | 40,00 | 40,00 |
| Propan/Butan 2,7 bar | 35,00 | 35,00 | 35,00 | 35,00 |

Herstellung:

Cyclomethicone und Isopropylmyristat werden vorgelegt und die Gele bzw. Siliciumdioxid unter Rühren solange gerührt, bis eine homogene Verteilung resultiert. Anschließend gibt man unter Rühren das Aluminiumchlorhydrat-Pulver hinzu, homogenisiert mit dem Ultra Turrax-Rührer für 30 sec. und entlüftet anschließend unter Vakuum. Zu der so entstandenen Paste gibt man die entsprechende Menge n-Pentan unter Rühren zu, füllt in Glasaerosolflaschen ab, verschließt diese und drückt das Propan-/Butan-Gemisch nach.

Das Sedimentationsverhalten kann sehr gut in den Glasaerosolflaschen beobachtet und beurteilt werden. Zu diesem Zweck werden die Flaschen 4mal vertikal kräftig geschüttelt und dann stehengelassen. Die Sedimentation wurde visuell an der sedimentierenden Grenzfläche fest/flüssig beurteilt. Dabei wird die Strecke vom Boden bis zur Gesamtfüllmenge von 100 ml ausgemessen und gleich 0 % Sedimentation gesetzt. Die Grenzfläche des sedimentierenden Feststoffs wird nach unterschiedlichen Zeiten ausgemessen und ins Verhältnis zu 0 % Sedimentation gesetzt.

Tabelle 4

| % sedimentierender Wirkstoff nach: | | | | | | |
|---|---|---|---|---|---|---|
|  | 0,5 min. | 1 min. | 5 min. | 10 min. | 20 min. | 60 min. |
| A | 0 % | 7 % | 21 % | 35 % | 50 % | 57 % |
| B | 0 % | 0 % | 7 % | 14 % | 28 % | 40 % |
| C | 33 % | 30 % | 58 % | 64 % | 65 % | 65 % |
| D | 100 % | 100 % | 100 % | 100 % | 100 % | 100 % |

Beurteilung:

Das Aerosol D ohne Antiabsetzmittel sedimentiert sofort und bildet einen festen Bodensatz. Das Aluminiumchlorhydrat-Pulver ist stark verklumpt und verstopft das Aerosolventil. Ein Versprühen des Aerosols ist nicht möglich.

Aerosol C sedimentiert ebenfalls sehr schnell, wogegen die Beispiele A und B sehr den Feststoff in der Schwebe halten. Der auf die Haut gesprühte Film zeigt im Gegensatz zu C ein deutlich weicheres Hautgefühl. Aerosol C neigt nach einiger Standzeit ebenfalls zu Ventilverstopfungen, während A und B ,die die erfindungsgemäße Sedimentierhilfe enthalten, sich vollständig und gleichmäßig entleeren lassen.

Alle diese Versuche zeigen, daß die neuen thermostabilen Lipogele erhebliche technische Vorteile gegenüber herkömmlichen Gelen zeigen.

Auf der Abbildung 1 ist das Röntgenbeugungsspektrum der Verbindung aus Beispiel 2 zu erkennen. Im allg. ist der dreidimensionale Aufbau der erfindungsgemäßen Al,Mg-Hydroxi-Verbindungen gekennzeichnet duch eine negativ geladene Zwischenschicht , die aus Anionen der unterschiedlichsten Art und Wasser bestehen kann und aus regelmäßig alternierenden Oktaederschichten, die aus den Kationen Al und Mg im Verhältnis 2:1 aufgebaut werden. Die Abfolge der Reflexe 003,006,009,018,110 und 113 ist ein sicheres Indiz für die Stapelung der Oktaederschichten.

Die Verbindungen gemäß der Erfindung sind durch die organisch-lipophile Verbindungen hydrophobiert.

Aus der Tabelle 5 kann die entsprechende Auswertung zum Spektrum auf der Abbildung 1 entnommen werden.

Tabelle 5

| Röntgenspektrum (Gerät:Phillips Automated X-Ray Diffractometer System APD 15 Verbindung nach Beispiel 2 | | | |
|---|---|---|---|
| Peak Nr. | 2-Theta | d (nm) | $I/I_0$ |
| 1 | 3,467 | 25,4602 | 98 |
| 2 | 5,155 | 17,1274 | 100 |
| 3 | 10,384 | 8,5121 | 25 |
| 4 | 10,624 | 8,3202 | 38 |
| 5 | 11,164 | 7,9184 | 75 |
| 6 | 19,962 | 4,4442 | 46 |
| 7 | 21,308 | 4,1662 | 17 |
| 8 | 21,909 | 4,0534 | 25 |
| 9 | 22,402 | 3,9653 | 33 |
| 10 | 22,599 | 3,9312 | 35 |

**Patentansprüche**

1. Aluminium,Magnesiumhydroxi-Fettsäure Verbindungen der allg. Formel

$$Al_2 Mg_y(OH)_{4+2y}R_{-2} \ n \ H_2O$$

in der R den Rest einer aliphatischen Monocarbonsäure $R'COO^-$ oder deren Mischungen darstellt, wobei $R'COO^-$ 12 bis 22 Kohlenstoffatome enthält und y folgende Bedingung erfüllt:

$$2 \leq y \leq 10$$

erhältlich durch die Umsetzung eines löslichen Aluminiumsalzes mit einem löslichen Magnesiumsalz im alkalischen Milieu bei einem pH-Wert von $\geq$ 8 und in Gegenwart eines Alkali-oder Ammoniumsalzes einer 12 bis 22 Kohlenstoffatome enthaltenden aliphatischen Monocarbonsäure oder deren Mischungen, wobei die wässerigen Lösungen der Aluminium und-Magnesiumsalze gleichzeitig in die alkalische Alkalicarbonsäurelösung bei Temperaturen von 20 bis 90 Grad C, bevorzugt 60 bis 85 Grad C unter intensivem Rühren zugegeben werden, wobei der pH-Wert stets $\geq$ 8 betragen muß, und der so erhaltene Niederschlag nach bekannten Methoden abgetrennt wird und mit vollentsalztem Wasser solange gewaschen wird, bis im Waschwasser keine Anionen der jeweils eingesetzten Aluminium und-Magnesiumsalze mehr nachweisbar sind.

2. Verbindungen nach Anspruch 1 dadurch gekennzeichnet, daß die Umsetzung unter Ausschluß von Fremdionen und unter Verwendung von vollentsalztem Wasser durchgeführt wird.

3. Verbindungen nach den Ansprüchen 1 und 2, dadurch gekennzeichnet, daß die wasserlöslichen Aluminiumverbindungen ausgewählt werden aus der Gruppe der Halogenide, Nitrate und Sulfate, sowie der basischen Aluminiumhalogenide oder Alkalialuminate.

**4.** Verbindungen nach den Ansprüchen 1 bis 3, dadurch gekennzeichnet, daß als Magnesiumkomponente die Halogenide, Nitrate und Sulfate von Magnesium eingesetzt werden.

**5.** Verbindungen nach Anspruch 1 bis 4, dadurch gekennzeichnet, daß die Einstellung des pH-Wertes während der Umsetzung mit Alkalihydroxiden oder $NH_4OH$ erfolgt.

**6.** Verbindungen nach Anspruch 1 bis 5, dadurch gekennzeichnet, daß während der Umsetzung die Anwesenheit von Kohlendioxid ausgeschlossen ist.

**7.** Thermostabile Lipogele, enthaltend eine Aluminium- Magnesiumhydroxi-Fettsäure Verbindung (Komponente a) nach Anspruch 1 und eine bei Raumtemperatur (20°C) flüssige, organische lipophile Verbindung (Komponente b), erhältlich durch die Umsetzung der Komponenten a und b unter intensivem Rühren bei Temperaturen zwischen 90 und 130 Grad C und unter einem Druck von mindestens 1 bar.

**8.** Thermostabile Lipogele nach Anspruch 7, dadurch gekennzeichnet, daß die organische, lipophile Verbindung mindestens eine Verbindung aus der Gruppe
    a) der pflanzlichen und tierischen Fette und Öle
    b) der Paraffinkohlenwasserstoffe,
    c) der Silikonöle,
    d) der aliphatischen und aromatischen Ester oder
    e) der höheren Alkohole und Ether, ist.

**9.** Thermostabile Lipogele nach den Ansprüchen 7 und 8, dadurch gekennzeichnet, daß die Konzentration der organischen lipophilen Verbindung 90 bis 70 Gew %., vorzugsweise 80 Gew. % beträgt.

**10.** Thermostabile Lipogele nach den Ansprüchen 7 bis 9, dadurch gekennzeichnet, daß die Konzentration der Aluminium, Magnesiumhydroxi-Fettsäure Verbindung nach Anspruch 1, 10 bis 30 Gew. %, bevorzugt 20 Gew.%, bezogen auf die thermo- und viskositätsstabilen Lipogele, beträgt.

**11.** Verwendung der neuen thermostabilen Lipogele nach den Ansprüchen 7 bis 10 in kosmetischen und pharmazeutischen Zubereitungen.

**Claims**

**1.** Aluminium, magnesium hydroxy fatty acid compounds of the general formula

$$Al_2Mg_y(OH)_{4+2y}R_{-2} \; n \; H_2O$$

in which R represents the radical of an aliphatic monocarboxylic acid $R'COO^-$ or mixtures thereof, wherein $R'COO^-$ contains 12 to 22 carbon atoms and fulfils the following condition:

$$2 \leq y \leq 10$$

obtainable by reacting a soluble aluminium salt with a soluble magnesium salt in alkaline medium at a pH value of $\geq 8$ and in the presence of an alkali metal or ammonium salt of an aliphatic monocarboxylic acid containing 12 to 22 carbon atoms or mixtures thereof, wherein the aqueous solutions of the aluminium and magnesium salts are added at the same time to the alkaline alkali metal carboxylic acid solution at temperatures of 20 to 90 degrees C, preferably 60 to 85 degrees C, with intensive stirring, wherein the pH value must always be $\geq 8$, and the precipitate thus obtained is separated off by known methods and washed using fully deionised water until anions of the particular aluminium and magnesium salts used can no longer be detected in the washing water.

**2.** Compounds according to claim 1, characterised in that the reaction is carried out with exclusion of foreign ions and using fully deionised water.

**3.** Compounds according to claims 1 and 2, characterised in that the water-soluble aluminium compounds are selected from the group comprising halides, nitrates and sulphates, and the basic aluminium halides or alkali metal aluminates.

4. Compounds according to claims 1 to 3, characterised in that the halides, nitrates and sulphates of magnesium are used as the magnesium component.

5. Compounds according to claim 1 to 4, characterised in that the pH value is adjusted during the reaction using alkali metal hydroxides or $NH_4OH$.

6. Compounds according to claim 1 to 5, characterised in that the presence of carbon dioxide is excluded during the reaction.

7. Thermostable lipogels containing an aluminium-magnesium hydroxy fatty acid compound (component a) according to claim 1 and an organic lipophilic compound which is liquid at room temperature (20°C) (component b), which can be obtained by the reaction of components a and b with intensive stirring at temperatures between 90 and 130 degrees C and under a pressure of at least 1 bar.

8. Thermostable lipogels according to claim 7, characterised in that the organic lipophilic compound is at least one compound from the group
   a) of vegetable and animal fats and oils,
   b) of paraffin hydrocarbons,
   c) of silicone oils,
   d) of aliphatic and aromatic esters, or
   e) of higher alcohols and ethers.

9. Thermostable lipogels according to claims 7 and 8, characterised in that the concentration of the organic lipophilic compound is 90 to 70 wt.%, preferably 80 wt.%.

10. Thermostable lipogels according to claims 7 to 9, characterised in that the concentration of the aluminium, magnesium hydroxy fatty acid compound according to claim 1 is 10 to 30 wt.%, preferably 20 wt.%, based on the thermostable and viscosity-stable lipogels.

11. Use of the novel thermostable lipogels according to claims 7 to 10 in cosmetic and pharmaceutical formulations.

**Revendications**

1. Composés d'hydroxyde d'aluminium et de magnésium et d'acide gras de formule générale

   $Al_2Mg_y(OH)4 + 2yR-_2nH_2O$

   dans laquelle R représente le radical d'un acide monocarboxylique aliphatique, $R'COO^-$ ou leurs mélanges tandis que $R'COO^-$ contient de 12 à 22 atomes de carbone et que y est conforme à la relation suivante :

   $2 \leqq y \leqq 10$

   pouvant être obtenus en faisant réagir un sel soluble de l'aluminium avec un sel soluble du magnésium en milieu alcalin à un pH ≧ 8 et en présence d'un sel de métal alcalin ou d'ammonium, d'un acide monocarboxylique aliphatique contenant de 12 à 22 atomes de carbone ou leurs mélanges tandis que les solutions aqueuses des sels d'aluminium et de magnésium sont ajoutées simultanément à la solution alcaline de sel alcalin d'acide carboxylique à des températures de 20 à 90°C et, de préférence, de 60 à 85°C avec agitation énergique, la valeur du pH devant toujours être ≧ 8, le précipité ainsi obtenu étant séparé par des méthodes connues en soi et lavé avec de l'eau complètement déminéralisée jusqu'à ce qu'aucun ion des sels d'aluminium et de magnésium utilisés ne puisse plus être décelé dans les eaux de lavage.

2. Composés selon la revendication 1, caractérisés en ce que la réaction est réalisée en l'absence d'ions étrangers et en utilisant de l'eau complètement déminéralisée.

11

3. Composés selon les revendications 1 et 2, caractérisés en ce que les composés de l'aluminium solubles dans l'eau sont choisis dans le groupe formé des halogénures, des nitrates et des sulfates ainsi que des halogénures basiques de l'aluminium ou des aluminates de métaux alcalins.

4. Composés selon les revendications 1 à 3, caractérisés en ce que l'on utilise comme composés du magnésium les halogénures, les nitrates et les sulfates de magnésium.

5. Composés selon les revendications 1 à 4, caractérisés en ce que le réglage de la valeur du pH est effectué pendant la réaction avec les hydroxydes alcalins ou avec $NH_4OH$.

6. Composés selon les revendications 1 à 5, caractérisés en ce que la présence de dioxyde de carbone est exclue au cours de la réaction.

7. Lipogels thermostables contenant un composé d'hydroxyde d'aluminium et de magnésium et d'acide gras (composant a) selon la revendication 1 et un composé lipophile organique liquide à température ambiante (20°C) (composant b) pouvant être obtenu en faisant réagir les composants a et b avec agitation énergique à des températures comprises entre 90 et 130°C et sous une pression d'au moins 1 bar.

8. Lipogels thermostables selon la revendication 7, caractérisés en ce que le composé lipophile organique est au moins un composé choisi dans le groupe formé des substances suivantes :
   a) les graisses et huiles végétales et animales,
   b) les hydrocarbures paraffiniques,
   c) les huiles de silicone,
   d) les esters aliphatiques et aromatiques ou
   e) les alcools et éthers supérieurs.

9. Lipogels thermostables selon les revendications 7 et 8, caractérisés en ce que la concentration du composé lipophile organique est comprise entre 90 et 70% en poids et est égale, de préférence, à 80% en poids.

10. Lipogels thermostables selon les revendications 7 à 9, caractérisés en ce que la concentration du composé d'hydroxyde d'aluminium et de magnésium et d'acide gras selon la revendication 1 est comprise entre 10 et 30% en poids et est égale, de préférence, à 20% en poids par rapport aux lipogels thermostables et à viscosité stable.

11. Utilisation des nouveaux lipogels thermostables selon les revendications 7 à 10 dans les préparations cosmétiques et pharmaceutiques.

Abbildung 1